# EUROPÄISCHE PATENTSCHRIFT

(11) **EP 2 217 180 B1**
(45) Veröffentlichungstag und Bekanntmachung des Hinweises auf die Patenterteilung: **13.04.2011**
(21) Anmeldenummer: 08849091.7
(22) Anmeldetag: 12.11.2008
(51) Int. Cl.: A61F 2/78, A61F 2/80

(54) **LINER FÜR VAKUUMSCHÄFTE UND VERWENDUNG DES LINERS**
LINER FOR VACUUM SOCKETS, AND USE OF THE LINER
MANCHON POUR EMBOÎTURES À FIXATION PAR LE VIDE ET SON UTILISATION

(30) Priorität: 13.11.2007 DE 202007015828 U
(43) Veröffentlichungstag der Anmeldung: 18.08.2010
(73) Patentinhaber: Otto Bock Healthcare GmbH, 37115 Duderstadt (DE)
(72) Erfinder: BIELEFELD, Thilo-Mathias, 29416 Siedenlangenbeck (DE)
(74) Vertreter: Lins, Edgar
(86) Internationale Anmeldenummer: PCT/DE2008/001874
(87) Internationale Veröffentlichungsnummer: WO 2009/062489

(56) Entgegenhaltungen:
- WO-A-01/21113
- WO-A-96/21405
- DE-U- 1 733 818
- US-A- 4 479 272
- US-A- 5 980 577
- US-A1- 2004 137 178

## Beschreibung

Die Erfindung betrifft Liner für Vakuumschäfte zur Aufnahme von Amputationsstümpfen zur Benutzung in prothetischen Schäften.

Die Erfindung betrifft ferner eine Verwendung eines derartigen Liners mit einem Vakuumschaft.

Bei Prothesen und insbesondere bei Beinprothesen ist man bemüht, eine größtmögliche Fläche am Amputationsstumpf zum Befestigen der Prothese zu realisieren. Das heißt, die auftretenden Kräfte auf eine größtmögliche Fläche zu verteilen, sowohl in Belastungs- als auch in der Schwungphase. Sogenannte Saugschäfte sind geeignet, größtmögliche Fläche zum Befestigen zu erreichen. Sie liegen luftdicht am Amputationsstumpf. Ist eine Kraft geneigt, die Prothese vom Stumpf zu ziehen, entsteht ein Unterdruck, der zusammen mit dem atmosphärischen Druck die Prothese am Amputationsstumpf hält. Beim Saugschaft ist eine einwandfreie Abdichtung zwingend notwendig, um die Fixierung zu gewährleisten. Ansonsten bricht der Unterdruck zusammen und der Amputationsstumpf rutscht aus dem Prothesenschaft.

Daraus ist ersichtlich, dass ein solches Ereignis zum Sturz des Prothesenträgers führen kann, weil die Prothese nicht mehr am Amputationsstumpf fixiert ist.

Aufgrund dessen, dass sich das Stumpfvolumen im Laufe eines Tages aufgrund von Temperatur, Blutdruck und sonstigen medizinischen Gründen deutlich verändern kann, ist eine hundertprozentige Abdichtung nicht immer gegeben. Daher kann es vorkommen, dass eine Prothese an einem Tag gut sitzt und an einem anderen Tag nicht: Diese Volumenschwankung kann sogar innerhalb von wenigen Stunden auftreten, in schwierigen Fällen ändert sich der Stumpf täglich. Daraus resultiert die schon genannte Gefahr.

Um dies zu verhindern, ist es üblich, einen Liner zu verwenden. Diese werden auf den Amputationsstumpf aufgerollt und sind an ihrem distalen Ende mit einem Fixierstift versehen, der in einen Rastmechanismus des Schaftes den Liner verriegelt. Aufgrund der Gasdichtheit und Reibungshaftung sitzt der Liner fest am Amputationsstumpf. Bei dieser Fixierungsanordnung kommt es zu einem sogenannten Melkeffekt wenn der Prothesenschaft auf Zug beansprucht wird, weil der Liner sich aufgrund seiner Nachgiebigkeit verjüngt. Marktübliche Vakuumliner beziehen nicht die komplette Stumpffläche für ein Vakuum zwischen Liner und Schaft mit ein.

US 2004/0137178 A1 offenbart einen Liner, der aus mehreren Schichten aufgebaut ist, wobei die äußere Schicht zur Reibungsverminderung durch ein Textil gebildet ist, das in eine Elastomerschicht eingebettet sein kann.

WO 96/21405 A1 offenbart eine prothetische Socke, die aus einem Textilmaterial, beispielsweise Baumwolle, besteht und über den Amputationsstumpf gezogen wird, um eine Polsterung zum Prothesenschaft zu gewährleisten. Zur Vermeidung einer relativen Bewegung der Socke zum Prothesenschaft ist die Socke mit mehreren reibungsvermindernden Flecken an ihrer Oberseite versehen.

US 4,479,272 offenbart eine aus mehreren Schichten aufgebaute elastische Hülse, die über einen Amputationsstumpf gezogen wird und einen Polsterungseffekt gegenüber dem Prothesenschaft bewirken soll. Die Lagen der Hülse sind Textillagen, die nicht für eine Abdichtung und Ausbildung eines Saugschafts vorgesehen sind. Die Lagen werden gemeinsam über den Rand des Schafts umgeschlagen, wobei der umgeschlagene Teil nochmals umgeschlagen werden kann, um eine axiale Fixierung der Hülse relativ zu dem Schaft zu gewährleisten. Hülse und Schaft sind dabei so ausgebildet, dass sie einer Beugung im Kniegelenk folgen können.

Eine ähnliche Schutzhülle ist durch DE 1 733 818 U bekannt. Zur Befestigung der elastischen Hülle für den Amputationsstumpf ist diese mit einer Befestigungsvorrichtung versehen, die aus einem elastischen Band am proximalen Teil der Hülle besteht. Das Band wird über eine Wollhülle umgeschlagen, woraufhin die Wollhülle und die Schutzhülle gemeinsam auf den oberen Teil der Prothese umgefaltet bzw. umgeschlagen wird.

Der Erfindung liegt die Aufgabe zugrunde, den Aufbau des Vakuums zwischen Liner und Schaftwand zu verbessern.

Zur Lösung dieser Aufgabe ist erfindungsgemäß ein Liner mit den Merkmalen des Patentanspruchs 1 versehen.

Der erfindungsgemäße Liner bietet den Vorteil, dass die komplette Schaftfläche für den Aufbau des Vakuums zwischen Liner und Schaftwand verwendbar ist, da die Abdichtung mit dem Außenliner erfolgt, während sich der Innenliner eng an dem Amputationsstumpf anpassen kann. Durch diese feste Ausbildung des Vakuums zwischen Liner und Schaftwand ist es möglich, auf eine distale Verriegelung mit einem Stahlstift und einem Rastmechanismus zu verzichten.

Die Erfindung soll im Folgenden anhand von in der Zeichnung dargestellten Ausführungsbeispielen näher erläutert werden. Es zeigen:
- Figur 1: ein erstes Ausführungsbeispiel eines erfindungsgemäßen Liners;
- Figur 2: ein zweites Ausführungsbeispiel eines erfindungsgemäßen Liners.

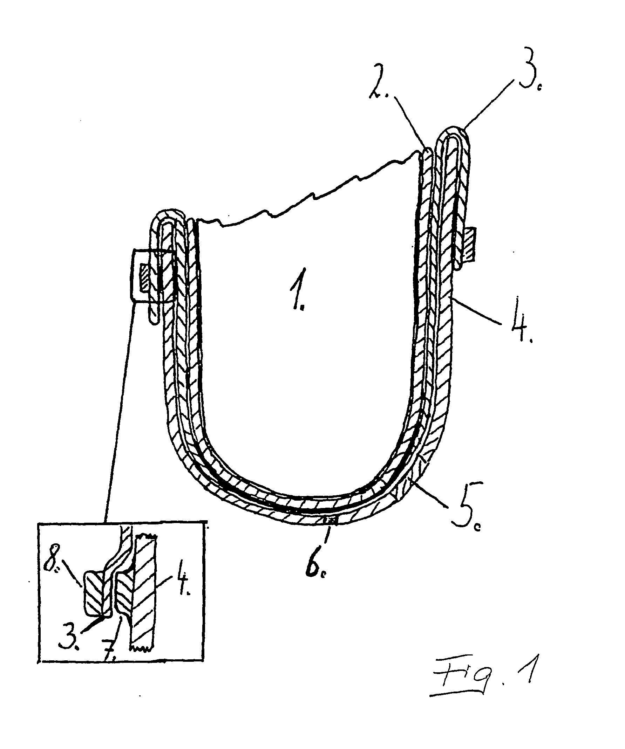

Der in Figur 1 dargestellte Liner besteht aus einem Innenliner 2, der im mittleren bis distalen Drittel mit einem Außenliner fest verbunden ist, wobei der Innenliner hierbei noch eine Standard-Ausgangslänge aufweist. Der Liner kann eine linerspezifische distale Gestaltung aufweisen, mit der ein Längshub verhindert wird, ein individueller distaler Cup eingesetzt ist und für eine notwendige Querdehnung gesorgt wird usw.

Zum Anlegen des Liners wird der komplette Liner auf links gedreht und der Innenliner 2 wird auf den Stumpf aufgerollt. Daraufhin kann der individuelle, proximale Verlauf der Kante des Innenliners festgelegt und der Innenliner entsprechend gekürzt werden. Der Außenliner 3 wird nun nach proximal umgeschlagen und der Amputationsstumpf 1 mit aufgerolltem Liner in den Schaft 4 eingeführt. Der Außenliner 3 wird nun über den Schaftrandverlauf 4 nach distal umgeschlagen und dichtet an der Außenseite des Schaftes 4 ab, um dadurch das Vakuum zwischen Schaft 4 und Liner 2, 3 über die gesamte Schaftinnenfläche zu realisieren. Der Liner wird dann individuell auf der Außenseite gekürzt. Um das Vakuum zu erhöhen, kann in den Schaft 4 ein handelsübliches Ausstoßventil 5 eingesetzt und/oder eine handelsübliche Vakuumpumpe über einen Anschluss 6 im Schaft 4 benutzt werden.

Als Option kann eine zusätzliche zirkuläre Verdickung 7 - im Sinne einer Dichtlippe auf der Außenseite des Prothesenschaftes angebracht werden, sie unterstützt die Abdichtung des Außenliners zum Schaftinneren. Zusätzlich kann noch eine zirkuläre mechanische Gurtung 8 von außen auf den Außenliner im Bereich der Dichtlippe angebracht werden, um die Dichtigkeit zu erhöhen.

Der sogenannte Melkeffekt wird deutlich reduziert und die auftretenden Kräfte werden über eine größere Fläche verteilt und damit pro Quadratzentimeter deutlich verringert. Das ganzheitliche Vakuum sorgt des Weiteren dafür, dass die Stumpfschwankungen stark reduziert oder sogar verhindert werden. Das bedeutet einerseits mehr Komfort beim Tragen der Prothese als auch längere Tragezeiten für Prothesenträger mit Stumpfvolumenschwankungen.

Der erfindungsgemäße Liner besteht vorzugsweise aus Silikon-Elastomeren, thermoplastischen Copolymer-Gelen oder Polyurethan-Gelen. Diese Materialien erlauben auch eine nachträgliche Veränderung der Höhe, bis zu der Innenliner 2 und Außenliner 3 miteinander verbunden sind, indem eine nachträgliche Verklebung von Innenliner und Auβenliner bis zu einer gewünschten Höhe vorgenommen wird. Der Liner kann ferner aus geeigneten Materialkombinationen gebildet sein.

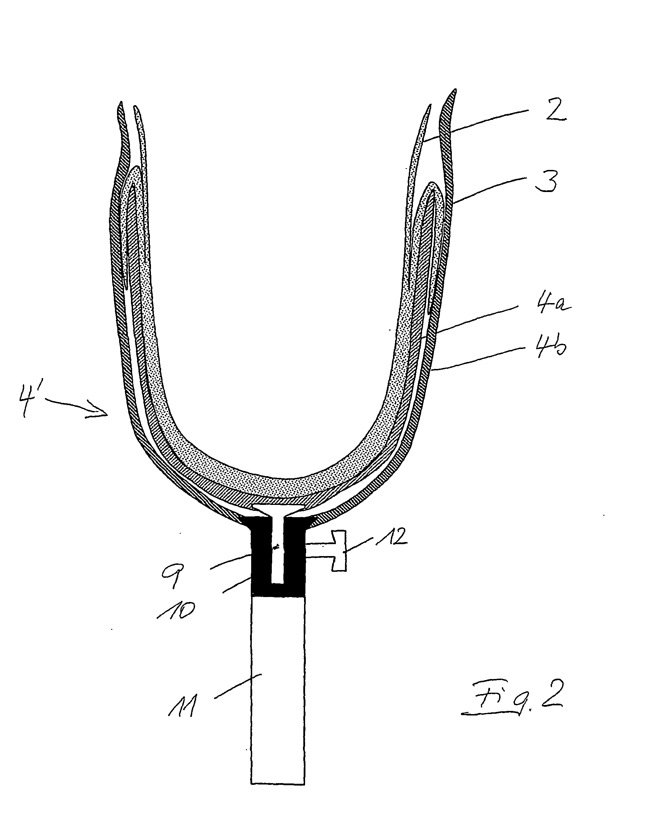

Der erfindungsgemäße Liner ist mit Vorteil mit einem doppelwandigen Schaft 4' verwendbar, wie er in Figur 2 dargestellt ist. Dabei kann der umgeschlagene Rand des Außenliners 3 in den Zwischenraum zwischen Schaftwänden 4a, 4b des doppelwandigen Schafts 4' hineinragen und dort abgedichtet sein. Mit Vorteil kann dabei die Innenseite des Außenliners 3 mit einem Textil oder mit einer reibungsmindernden Schicht beschichtet sein. Insbesondere im umgeschlagenen Bereich des Außenliners 3 wird dieser durch die aufgebrachte Textillage oder reibungsmindernde Schicht gegen Verschleiß geschützt, insbesondere auch bei dem doppelwandigen Prothesenschaft 4'.

Selbstverständlich kann der Liner 2, 3 auch an seiner Außenseite mit einer reibungsmindernden Schicht oder einem Textil beschichtet sein, um die Einführung in den Prothesenschaft 4 zu erleichtern. Da die Beschichtung die Dichtwirkung des Außenliners 3 im Bereich des umgeschlagenen Randes behindern kann, ist es zweckmäßig, wenn die Beschichtung der Außenseite deutlich unter der Höhe des Prothesenschafts 4 endet, beispielsweise an der Linie, bis zu der Innenliner 2 und Außenliner 3 miteinander verbunden sind.

Wie Figur 2 verdeutlicht ist eine Innenwand 4a des Schafts 4' nach proximal kürzer ausgebildet als die Außenwand 4b. Die Außenwand 4b ist darüber hinaus im Wesentlichen starr, während die Innenwand 4a zwar nicht dehnbar, jedoch leicht verformbar ist, sodass eine Anpassung an eine unregelmäßige Form des Amputationsstumpfes 1 möglich ist. Die Innenwand 4a weist am distalen Ende einen integrierten Verriegelungsbolzen 9 auf, der mit einem entsprechenden Schloss 10 am distalen Ende der Außenwand 4b zur Herstellung einer Verbindung zusammenwirkt. Das Schloss 10 befindet sich an einem Modularrohr 11. Die Verbindung zwischen Verriegelungsbolzen 9 und Schloss 10 kann mit einer Entriegelungstaste 12 gelöst werden. Bei einem doppelwandigen Schaft 4' hat die Außenwand 4b ausschließlich eine stützende Funktion, sodass die Außenwand 4b nicht durchgehend ausgebildet sein muss. Es ist daher auch möglich, eine stützende Rahmenkonstruktion als Außenwand 4b vorzusehen.

## Patentansprüche

1. Liner für Vakuumschäfte zur Aufnahme von Amputationsstümpfen in prothetischen Schäften (4), wobei der Liner doppelwandig aus einem Innenliner (2) und einem Außenliner (3) ausgebildet ist, **dadurch gekennzeichnet, dass** der Innenliner (2) und der Außenliner (3) im mittleren und/oder distalen Drittel fest miteinander verbunden sind und der Außenliner (3) zur Abdichtung mit dem prothetischen Schaft (4) durch Umschlagen über den Schaftrandverlauf nach distal ausgebildet ist, während der Innenliner (2) bis zu seiner proximalen Kante am Amputationsstumpf anliegt.

2. Liner nach Anspruch 1, **dadurch gekennzeichnet, dass** der Außenliner (3) ringförmig an dem Innenliner (2) in Richtung proximal angesetzt ist.

3. Verwendung des Liners nach Anspruch 1 oder 2, bei der die Abdichtung durch den Außenliner (3) am Rand oder an der Außenseite des prothetischen Schafts (4) erfolgt.

4. Verwendung nach Anspruch 3, bei der der Außenliner (3) mit einem am proximalen Ende nach distal umgeschlagenen Rand an der Außenseite des prothetischen Schafts (4) anliegt.

5. Verwendung nach Anspruch 4, bei der zur Abdichtung eine außen am prothetischen Schaft (4) gasdicht befestigte Dichtlippe (7) vorgesehen ist.

6. Verwendung nach Anspruch 5, bei der der Anpressdruck des Auβenliners (3) auf der Höhe der Dichtlippe (7) durch eine zirkuläre Gurtung (8) verstärkt wird.

7. Verwendung des Liners nach Anspruch 1 oder 2 mit einem prothetischen Schaft (4), der doppelwandig mit einer Innenwand und einer Außenwand ausgebildet ist, wobei der Außenliner (3) über den Rand der Innenwand umgeschlagen ist und in den Zwischenraum zwischen den beiden Wänden des prothetischen Schafts (4) ragt.

## Claims

1. Liner for vacuum sockets for receiving amputation stumps and for use in prosthetic sockets (4), said liner having a double wall made up of an inner liner (2) and of an outer liner (3), **characterized in that** the inner liner (2) and the outer liner (3) are fixedly connected to each other in the middle third and/or distal third, and the outer liner (3) is designed to provide a seal with the prosthetic socket (4) by being turned back in the distal direction over the socket edge profile, while the inner liner (2) bears up to its proximal edge on the amputation stump.

2. Liner according to Claim 1, **characterized in that** the outer liner (3) is fitted annularly on the inner liner (2) in the proximal direction.

3. Use of the liner according to Claim 1 or 2, in which the seal provided by the outer liner (3) is at the edge or on the outside of the prosthetic socket (4).

4. Use according to Claim 3, in which the outer liner (3) bears on the outside of the prosthetic socket (4) via an edge that is turned back in the distal direction at the proximal end.

5. Use according to Claim 4, in which a sealing lip (7) secured in a gastight manner on the outside of the prosthetic socket (4) is provided for the sealing.

6. Use according to Claim 5, in which the contact pressure of the outer liner (3) at the height of the sealing lip (7) is increased by a circular binding (8).

7. Use of the liner according to Claim 1 or 2 with a prosthetic socket (4) that has a double wall made up of an inside wall and of an outside wall, wherein the outer liner (3) is turned back over the edge of the inside wall and protrudes into the space between the two walls of the prosthetic socket (4).

## Revendications

1. Manchon pour emboîtures à fixation par le vide pour recevoir des moignons d'amputation dans des emboîtures de prothèse (4), le manchon étant formé à double paroi par un manchon interne (2) et un manchon externe (3), **caractérisé en ce que** le manchon interne (2) et le manchon externe (3) sont fermement reliés ensemble dans le tiers médian et/ou distal et **en ce que** le manchon externe (3) est formé pour l'étanchéité avec l'emboîture de prothèse (4) par rabat en direction distale sur le tracé du bord d'emboîture, tandis que le manchon interne (2) repose jusqu'à son bord proximal sur le moignon d'amputation.

2. Manchon selon la revendication 1, **caractérisé en ce que** le manchon externe (3) est mis en place en forme d'anneau sur le manchon interne (2) dans la direction proximale.

3. Utilisation du manchon selon la revendication 1 ou 2, dans laquelle l'étanchéité est assurée par le manchon externe (3) sur le bord ou sur la face externe de l'emboîture de prothèse (4).

4. Utilisation selon la revendication 3, dans laquelle le manchon externe (3) est adjacent à la face externe de l'emboîture de prothèse (4), par un bord à l'extrémité proximale, rabattu en direction distale.

5. Utilisation selon la revendication 4, dans laquelle il est prévu, pour l'étanchéité, une lèvre d'étanchéité (7) fixée hermétiquement au gaz, extérieurement sur l'emboîture de prothèse (4).

6. Utilisation selon la revendication 5, dans laquelle la pression d'appui du manchon externe (3) au niveau de la lèvre d'étanchéité (7) est renforcée par une courroie (8) circulaire.

7. Utilisation du manchon selon la revendication 1 ou 2 avec une emboîture de prothèse (4) qui est formée à double paroi avec une paroi interne et une paroi externe, le manchon externe (3) étant rabattu sur le bord de la paroi interne et s'engage dans l'interstice entre les deux parois de l'emboîture de prothèse (4).
